# EUROPEAN PATENT SPECIFICATION

(11) **EP 4 119 105 B1**
(45) Date of publication and mention of the grant of the patent: **07.05.2025**
(21) Application number: 21767297.1
(22) Date of filing: 09.03.2021
(51) Int. Cl.: A61F 2/89, A61F 2/90

(54) **STENT**
STENT
STENT

(30) Priority: 12.03.2020 JP 2020043037
(43) Date of publication of application: 18.01.2023
(73) Proprietor: JMS Co., Ltd., Hiroshima-shi Hiroshima 730-8652 (JP)
(72) Inventor: YAMAMOTO Ayaka, Hiroshima-shi, Hiroshima 730-8652 (JP); FUKUTAKI Shuji, Hiroshima-shi, Hiroshima 730-8652 (JP); NISHIHARA Manami, Hiroshima-shi, Hiroshima 730-8652 (JP)
(74) Representative: HGF
(86) International application number: PCT/JP2021/009327
(87) International publication number: WO 2021/182475

(56) References cited:
- EP-B1- 0 918 496
- EP-B1- 1 909 710
- WO-A1-2020/196911
- DE-A1- 10 103 000
- JP-A- 2019 155 178
- JP-A- 2019 520 116

## Description

### TECHNICAL FIELD

The present invention relates to a stent.

### BACKGROUND ART

Conventionally, in a stenosis disease (tumor, inflammation, or the like) in a biological tract such as a blood vessel or a gastrointestinal tract, a stent is placed in a stenosed site to expand the stenosed site. As a stent, for example, a stent made of metal or a synthetic resin is known. For example, there is known a stent formed by connecting a plurality of polygonal annular portions each formed of a linear member and in a polygonal annular shape when viewed in an axial direction, in a state of being bent or curved in the axial direction, side by side in the axial direction of the stent (for example, see Patent Document 1). EP 1909710 B1 discloses a stent that is positioned in a radially contracted position inside a duct, then expands until it reaches a size suitable for the duct.

Patent Document 1: Japanese Patent No.5384359

### DISCLOSURE OF THE INVENTION

### Problems to be Solved by the Invention

With regard to the stent disclosed in Patent Document 1, adjacent polygonal annular portions are joined to each other by passing the connecting parts of the adjacent polygonal annular portions through a short cylindrical tube through and fixing the tube part with an adhesive or the like. In this case, since the tube is formed in a cylindrical shape in the connecting parts of the adjacent polygonal annular portions, the stent becomes bulky in a radial direction, and thus it is difficult to store the stent in a delivery system.

It is an object of the present invention to provide a stent having improved storability in a delivery system.

### Means for Solving the Problems

The present invention relates to a stent formed of a linear member and in a cylindrical shape having a plurality of openings. The stent extends in an axial direction and is deformable from a reduced diameter state to an expanded diameter state. The stent is formed by connecting a plurality of polygonal annular portions each formed in a polygonal annular shape when viewed in an axial direction, in a state of being bent or curved in the axial direction, side by side in the axial direction of the stent. The linear members constituting the polygonal annular portions adjacent to each other in the axial direction respectively has through holes passing through in the axial direction. The linear members are connected to each other with a joining member passing through two of the through holes of the linear members constituting the polygonal annular portions adjacent to each other.

The present invention relates to a stent formed of a linear member and in a cylindrical shape having a plurality of openings. The stent extends in an axial direction and is deformable from a reduced diameter state to an expanded diameter state. The stent is formed by connecting a plurality of polygonal annular portions each formed in a polygonal annular shape when viewed in an axial direction, in a state of being bent or curved in the axial direction, side by side in the axial direction. Each of the polygonal annular portions is formed in an annular shape by joining one end part to the other end part of the linear member. The one end part and the other end part of each of the polygonal annular portions respectively have through holes passing through in the axial direction. The one end part and the other end part are connected to each other with a joining member passing through two of the through holes of the linear members constituting the polygonal annular portions adjacent to each other.

The joining member preferably passes through the two through holes and extends in the axial direction.

At least one selected from the linear member constituting the stent and the joining member is preferably formed of a biodegradable fiber.

The joining members preferably extend linearly and are respectively provided at a plurality of locations apart from each other in a circumferential direction of the stent. One of the joining members preferably joins the linear members constituting the polygonal annular portions adjacent to each other in the axial direction of the stent by passing through the two through holes and crossing. The two through holes are preferably joined to each other at each of a plurality of locations along the axial direction of the stent.

The through holes are preferably formed with a needle or a laser.

The joining member is preferably formed in a linear shape or a ring shape.

### Effects of the Invention

According to the present invention, it is possible to provide a stent having improved storability in a delivery system.

### BRIEF DESCRIPTION OF THE DRAWINGS

FIG. 1 is a perspective view showing a stent according to a first embodiment of the present invention;
FIG. 2 is an exploded view of the stent according to the present embodiment;
FIG. 3 shows a joined state of adjacent polygonal annular portions;
FIG. 4 shows a joined state of one end part and the other end part of a polygonal annular portion;
FIG. 5A shows a joining member according to a first modification.
FIG. 5B shows a joining member according to a second modification;
FIG. 5C shows a joining member according to a third modification;
FIG. 6 is a perspective view showing a stent according to a second embodiment;
FIG. 7 shows a linear joining member joining at a plurality of locations along the axial direction of the stent;
FIG. 8 shows a joined state of the linear joining member which joins at a predetermined location at an intermediate side in the axial direction of the stent;
FIG. 9 shows a joined state of the linear joining member joining at an end side in the axial direction of the stent; and
FIG. 10 shows a modification of the second embodiment and a joined state of a linear joining member joining at an end side in the axial direction of a stent.

### PREFERRED MODE FOR CARRYING OUT THE INVENTION

### (First Embodiment)

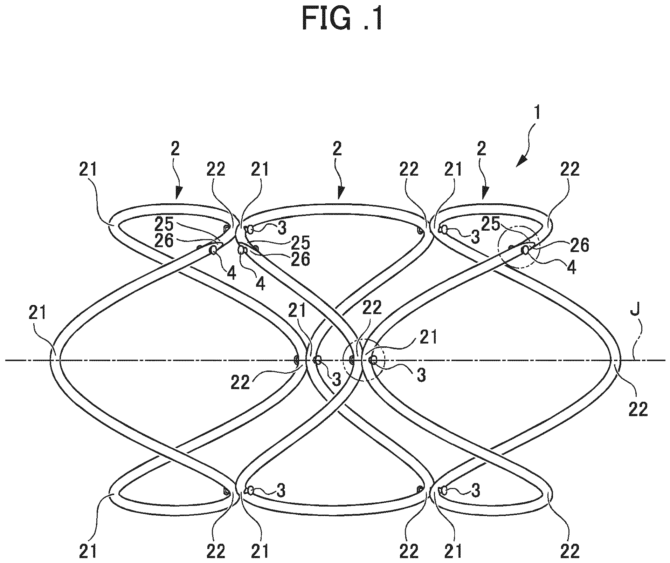

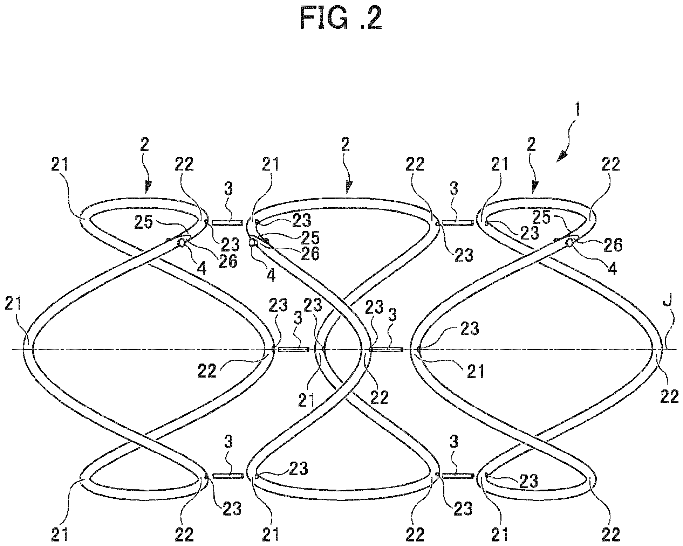

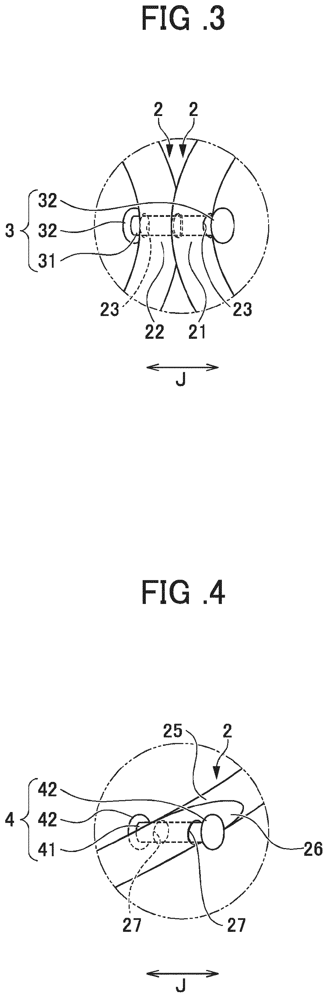

Hereinafter, a stent according to a first embodiment of the present invention will be described with reference to the drawings. In the description of the present embodiment, the direction in which a stent 1 extends as a whole is referred to as an axial direction J. FIG. 1 is a perspective view showing the stent 1 according to the first embodiment of the present invention. FIG. 2 is an exploded view of the stent 1 according to the present embodiment. FIG. 3 shows a joined state of adjacent polygonal annular portions 2. FIG. 4 shows a joined state of one end part 25 and the other end part 26 of a polygonal annular portion 2.

The stent 1 of the present embodiment is formed of biodegradable fibers (linear members) and has a plurality of openings. As shown in FIG. 1, the stent 1 is formed in a cylindrical shape extending in the axial direction J as a whole, and is deformable between a reduced diameter state (extended in the axial direction J) and an expanded diameter state (contracted in the axial direction J).

As shown in FIG. 2, the stent 1 is formed in a cylindrical shape having a plurality of openings by connecting a plurality of polygonal annular portions 2 side by side in the axial direction J. Each of the plurality of polygonal annular portions 2 is formed of a synthetic resin fiber and in an annular shape. The polygonal annular portion 2 has bent parts 21 and 22 formed by alternately bending toward one side and toward the other side in the axial direction J, in the middle of a circumferential direction. The bent parts 21 and 22 respectively constitute mountain parts and valley parts of the polygonal annular portion 2. In the present embodiment, the number of the mountain parts of the polygonal annular portion 2 is, for example, three. The bent part 21 is formed to be convex toward one side (the left side in FIGS. 1 and 2) in the axial direction J, and the bent part 22 is formed to be convex toward the other side (the right side in FIGS. 1 and 2) in the axial direction J.

The polygonal annular portions 2 adjacent to each other in the axial direction are connected to each other in the axial direction J with a joining member 3. The adjacent polygonal annular portions 2 are connected with the joining member 3 at the bent parts 21 and 22, which are convex on the side where they approach each other in the axial direction.

As shown in FIG. 3, through holes 23 passing through in the axial direction J are respectively formed in the bent parts 21 and 22 of the biodegradable fibers (linear members) constituting the polygonal annular portions 2 adjacent to each other in the axial direction J. The through hole 23 is formed in the biodegradable fiber with, for example, a needle, a laser, or the like. The shape of the through hole 23 is, for example, circular or quadrangular. A joining member linear part 31 of a linear joining member 3 is inserted into the through hole 23, and processed end parts 32 formed at both ends in the direction in which the joining member linear part 31 extends, are processed by melting with heat. Thus, the processed end part 32 has a larger diameter than the through hole 23. The joining member 3 passes through the through holes 23 and extends in the axial direction J. The joining member 3 passes through the two through holes 23 of the biodegradable fibers constituting the adjacent polygonal annular portions 2.

As shown in FIGS. 1 and 2, the biodegradable fiber (linear member) constituting each of the polygonal annular portions 2 is formed in an annular shape around the central axis extending in the axial direction J by joining the one end part 25 and the other end part 26. In the present embodiment, the one end part 25 and the other end part 26 of the biodegradable fiber constituting the polygonal annular portion 2 are cut at an angle to the direction in which the biodegradable fiber extends, and are joined by overlapping the oblique cut surfaces with each other. The one end part 25 and the other end part 26 of the biodegradable fiber constituting the polygonal annular portion 2 may not be cut at an angle to the direction in which the biodegradable fiber extends, but may be cut orthogonal to the direction in which the biodegradable fiber extends. In this case, the one end part 25 and the other end part 26 may be disposed so as to overlap each other in the axial direction J.

As shown in FIG. 4, through holes 27 passing through in the axial direction J are formed in the one end part 25 and the other end part 26 of the biodegradable fiber (linear member) constituting the polygonal annular portion 2. A joining member linear part 41 of a linear joining member 4 is inserted into the through holes 27, and processed end parts 42 formed at both ends in the direction in which the joining member linear part 41 extends are processed by melting with heat. Thereby, the processed end part 42 has a larger diameter than the through hole 27. The joining member 4 passes through the through holes 27 and extends in the axial direction J. The joining member 4 passes through the two through holes 23 of the biodegradable fibers constituting the adjacent polygonal annular portions 2.

The materials of the linear member and the joining members 3 and 4 constituting the polygonal annular portion 2 are not limited, but, for example, in the present embodiment, a biodegradable resin is used. As the biodegradable resin, for example, polylactic acid (PLA) or polydioxanone (PDO) is used. The materials of the linear member constituting the polygonal annular portion 2 and the joining members 3 and 4 are not limited thereto, and for example, a non-biodegradable resin or a biodegradable metal such as magnesium may be used.

The fiber diameter of the synthetic resin fiber constituting the stent 1 is, for example, 0.05 mm to 0.7 mm, preferably 0.4 mm to 0.6 mm. When the fiber diameter of the synthetic resin fiber constituting the stent 1 is, for example, 0.4 mm to 0.6 mm, the hole diameter of the through hole 23 is preferably 0.2 mm to 0.3 mm. The size of the stent 1 is not limited, but, for example, the stent 1 has a diameter of 10 to 25 mm and a length of 30 to 250 mm in a state where the stent 1 is expanded in diameter.

In the present embodiment, for example, the following stent 1 is made. The stent 1 is made by using PDO fibers (fiber diameter: 0.4 mm to 0.6 mm) and connecting three polygonal annular portions 2 in the axial direction J. Here, a round through hole 23 is formed with a needle having a diameter of 0.2 mm in each of the bent parts 21 and 22 of the PDO fiber (fiber diameter: 0.4 mm to 0.6 mm). In this case, the measured diameter of the through hole 23 is 0.197 mm. Each of the polygonal annular portions 2 is joined to each other by using a joining member 3 made of a PLA fiber (fiber diameter: 0.2 mm) in the through holes 23 formed in the bent parts 21 and 22. After the joining member 3 made of the PLA fiber (fiber diameter: 0.2 mm) is inserted into the through hole 23, both end parts of the joining member 3 are processed by melting with heat to prevent the joining member 3 from coming out of the through hole 23.

Forming the through hole 23 is not limited to forming the through hole 23 with a needle, and a laser may be used to form the through hole 23. In this case, a PDO fiber (fiber diameter: 0.56 mm) is used, and the through hole 23 is formed in the bent parts 21 and 22 made of the PDO fiber (fiber diameter: 0.56 mm) with a laser. The average of the measured diameters of the through holes 23 (the average of six locations) is 0.186 mm.

According to the stent 1 of the present embodiment described above, the following effects can be achieved.
(1) The stent 1 is formed by connecting the plurality of polygonal annular portions 2, each formed of a biodegradable fiber and in a polygonal annular shape when viewed in the axial direction J, side by side in the axial direction J of the stent 1, in a state of being bent or curved in the axial direction J. The biodegradable fibers constituting the polygonal annular portions 2 adjacent to each other in the axial direction J are respectively configured to have through holes 23 passing through in the axial direction J, and the polygonal annular portions 2 are connected to each other with the joining member 3 passing through the through holes 23 and extending in the axial direction J. Thus, in the through holes 23 passing through in the axial direction J, the adjacent polygonal annular portions 2 are joined to each other with the joining member 3 extending in the axial direction J, whereby the radial bulkiness of the stent 1 can be reduced. Therefore, for example, when the stent 1 is placed in a gastrointestinal tract with a stent delivery system, it is possible to improve the storability in the stent delivery system. Further, by improving the storability in the delivery system, it is possible to use a fiber having a larger diameter and to improve the strength of the stent 1. Further, since a fiber having a larger diameter can be used, the diameter of the through hole 23 can be increased. In addition, since the adjacent polygonal annular portions 2 are not fixed with a tube, an adhesive, or the like, the fibers can move in a flexible manner in the joining parts of the plurality of polygonal annular portions 2, so that it is possible to improve the followability of the peristaltic motion to the gastrointestinal tract.
(2) The stent 1 is formed by connecting the plurality of polygonal annular portions 2 each formed of a biodegradable fiber and in a polygonal annular shape when viewed in the axial direction J, side by side in the axial direction J of the stent 1, in a state of being bent or curved in the axial direction J. The polygonal annular portion 2 is formed in an annular shape by joining the one end part 25 to the other end part 26 of the biodegradable fiber, and the one end part 25 and the other end part 26 of the polygonal annular portion 2 are each configured to have the through hole 27 passing through in the axial direction J, and are connected to each other with the joining member 4 passing through the through holes 27 and extending in the axial direction J. Thus, for example, when the stent 1 is placed in a gastrointestinal tract with a stent delivery system, with regard to the stent 1 of the present embodiment, the one end part 25 and the other end part 26 of the polygonal annular portion 2 are joined to each other with the joining member 4 in the through holes 27 passing through in the axial direction J, so that the radial bulkiness of the stent 1 can be reduced, and the storability in the stent delivery system can be improved. The improvement of the storability in the stent delivery system enables a fiber having a larger diameter to be used and the strength of the stent 1 to be improved. Further, since a fiber having a larger diameter can be used, the diameter of the through hole 27 can be increased.
(3) At least one of the linear member constituting the stent 1 and the joining members 3 and 4 is formed of a biodegradable fiber. Thus, by connecting the plurality of polygonal annular portions 2 side by side in the axial direction J with the joining members 3, the timing at which the linear members or the joining members 3 and 4 are broken to disassemble the stent 1 can be controlled. Therefore, the stent 1 can be disassembled at a more intended timing.

### (Modifications)

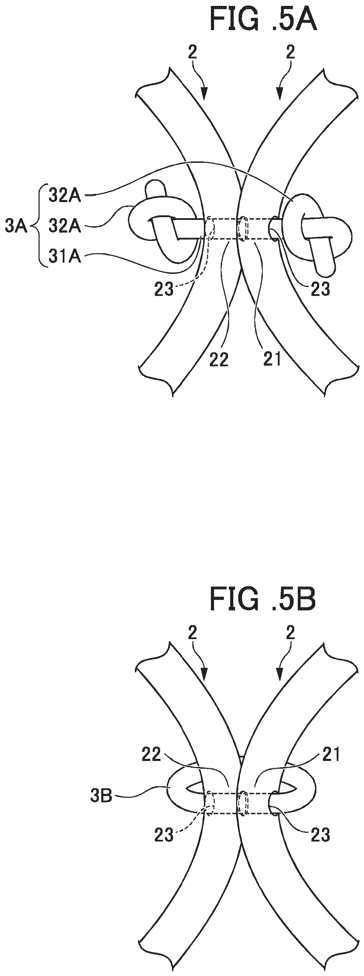

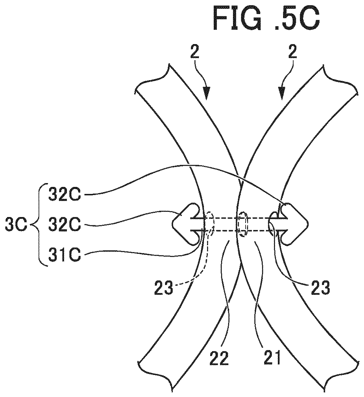

Modifications of the joining member will be described. FIG. 5A shows a joining member 3A of a first modification. FIG. 5B shows a joining member 3B of a second modification. FIG. 5C shows a joining member 3C of a third modification. With regard to the joining members 3A, 3B, and 3C of the modifications, the case in which the polygonal annular portions 2 are connected to each other in the axial direction J will be described, but the same applies to the case in which the one end part 25 and the other end part 26 of the polygonal annular portion 2 are connected to each other in the axial direction J in the above-described embodiment.

As shown in FIG. 5A, the joining member 3A of the first modification is configured by forming knots 32A and 32A at both end parts of a linear joining member main body 31A. As shown in FIG. 5B, the joining member 3B of the second modification is formed in a ring shape in a state of passing through the through hole 23. As shown in FIG. 5C, the joining member 3C of the third modification is configured in the following manner: Folded parts 32C and 32C are formed at both end parts of a linear joining member main body 31C. The folded parts 32C and 32C are folded and the joining member main body 31C is inserted into the through hole 23 without processing the both end parts of the joining member main body 31C. Then, the folded part 32C is spread so as to catch the biodegradable fiber (linear member) constituting the polygonal annular portion 2. According to the joining member 3A of the first modification, the joining member 3B of the second modification, and the joining member 3C of the third modification, the same effects as those of the above-described embodiment can be achieved.

Although a preferred embodiment of the stent of the present invention has been described above, the present invention is not limited to the above-described embodiment and can be as appropriate modified.

For example, in the above embodiment, biodegradable fibers are used as the linear members of the stent, but the present invention is not limited thereto. That is, non-biodegradable synthetic resin fibers may be used to construct the stent. The stent may be formed of a biodegradable metallic material such as magnesium as the linear member of the stent.

In the above embodiment, the stent 1 formed by connecting the plurality of polygonal annular portions 2 side by side in the axial direction J has been described. Here, in the present invention, the phrase 'the stent is formed by connecting a plurality of polygonal annular portions side by side in an axial direction' means that the stent is formed to have a shape in which a plurality of polygonal annular portions are arranged in the axial direction. For example, as in the above embodiment, a plurality of polygonal annular portions may be separately made and formed side by side in the axial direction, and may be connected to each other with joining members extending in the axial direction at locations where connection is required. Alternatively, the present invention is not limited to the configuration of the above embodiment, for example, a plurality of polygonal annular portions may be formed side by side by knitting one or more linear members in a spiral shape, and connected to each other with joining members extending in the axial direction at locations where connection is required. The number of the linear members is not limited.

In the above embodiment, the stent 1 in which three polygonal annular portions 2 are connected side by side in the axial direction J has been described, but the present invention is not limited thereto. For example, the stent 1 may be formed by connecting four or more polygonal annular portions 2 side by side in the axial direction J. The number of corners of the polygonal annular portion 2 is not limited.

### (Second Embodiment)

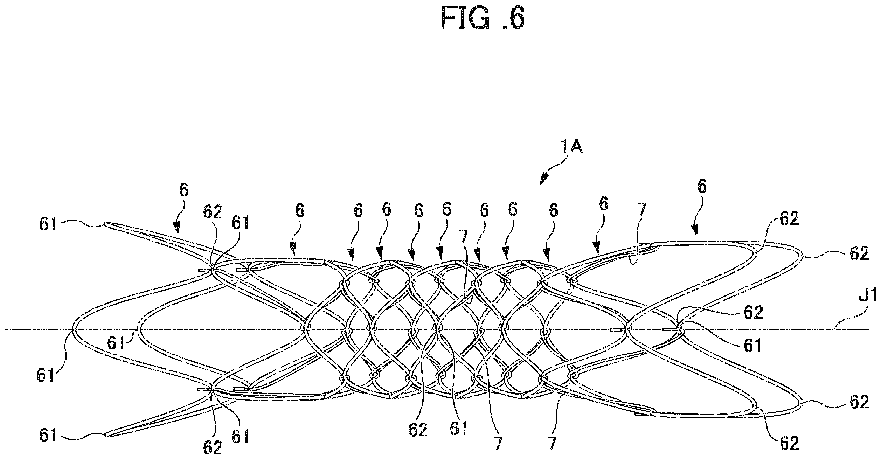

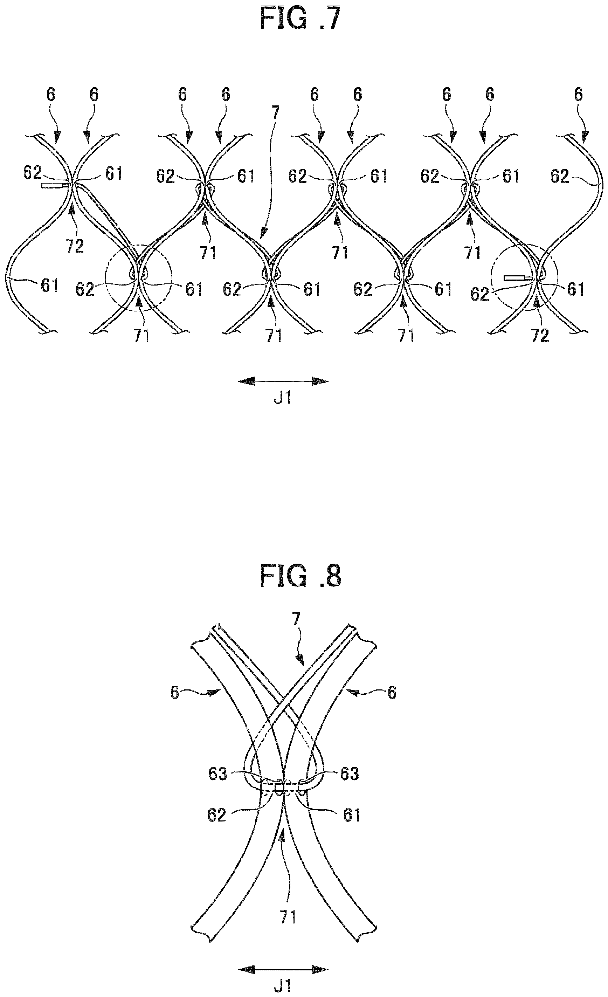

A stent 1A of a second embodiment will be described. The description of the first embodiment can be referred to for portions not described in the second embodiment. The stent 1A of the present embodiment is formed of biodegradable fibers (linear members) and has a plurality of openings. As shown in FIGS. 6 and 7, the stent 1A is formed in a cylindrical shape extending in an axial direction J1 as a whole, and is deformable between a reduced diameter state (extended in the axial direction J1) and an expanded diameter state (contracted in the axial direction J1).

The stent 1A is formed in a cylindrical shape having a plurality of openings by connecting a plurality of polygonal annular portions 6 side by side in the axial direction J1. Each of the plurality of polygonal annular portions 6 is formed of a synthetic resin fiber and in an annular shape. The polygonal annular portion 6 has bent parts 61 and 62 formed by alternately bending one side and the other side in the axial direction J1 in the middle of a circumferential direction. The bent parts 61 and 62 respectively constitute mountain and valley parts of the polygonal annular portion 6. The bent part 61 is formed to be convex toward one side (the left side in FIG. 6) in the axial direction J1, and the bent part 62 is formed to be convex toward the other side (the right side in FIG. 6) in the axial direction J1.

The polygonal annular portions 6 adjacent to each other in the axial direction are connected to each other in the axial direction J1 with a plurality of linear joining members 7 (joining members) at bent parts 61 and 62 which are convex toward each other in the axial direction.

Each of the plurality of linear joining members 7 is formed of one linear member and joins two through holes 63 at each of a plurality of locations along the axial direction of the stent 1A. Each of the linear joining members 7 formed of one linear member linearly extends in the axial direction of the stent 1A in a zigzag shape in which the circumferential position of the stent 1A is alternately shifted to one side and the other side. The plurality of linear joining members 7 are provided apart from each other in the circumferential direction of the stent 1A.

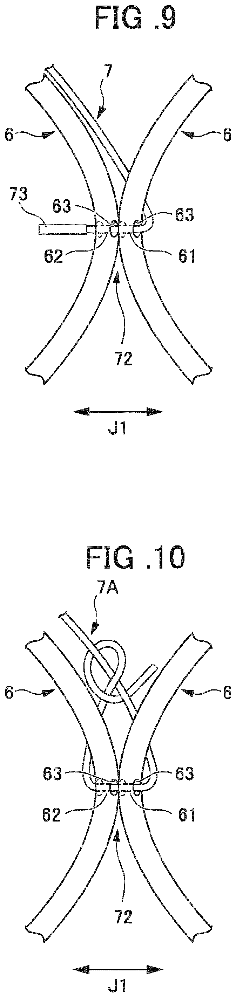

As shown in FIGS. 8 and 9, in the bent parts 61 and 62, the through holes 63 passing through in the axial direction J1 are respectively formed in the biodegradable fibers constituting the polygonal annular portions 6 adjacent to each other in the axial direction J1. The through hole 63 is formed in the biodegradable fiber with, for example, a needle, a laser, or the like. The shape of the through hole 63 is, for example, circular or quadrangular. The linear joining member 7 is inserted into the through hole 63.

As shown in FIG. 7, each of the plurality of linear joining members 7 joins the biodegradable fibers constituting the polygonal annular portions 6 adjacent to each other in the axial direction, at a plurality of intermediate joining parts 71 disposed on the inner side of both axial ends of the stent 1A, and joins the polygonal annular portions 6 adjacent to each other in the axial direction, at end side joining parts 72 disposed on both axial end sides of the stent 1A.

More specifically, as shown in FIG. 8, at the intermediate joining part 71, the linear joining member 7 passes through the two through holes 63 of the biodegradable fibers constituting the adjacent polygonal annular portions 6 and crosses, thereby joining the biodegradable fibers constituting the polygonal annular portions 6 adjacent to each other in the axial direction. The linear joining member 7 extends in the axial direction at the part of the intermediate joining part 71 in which the linear joining member 7 passes through the two through holes 63.

As shown in FIG. 9, at the end side joining part 72, the linear joining member 7 passes through the two through holes 63 of the biodegradable fibers constituting the adjacent polygonal annular portions 6, and joins the biodegradable fibers constituting the polygonal annular portions 6 adjacent to each other in the axial direction by fixing a tube 73 having an outer diameter larger than that of the through hole 63 to an end part of the linear joining member 7 with an adhesive. The linear joining member 7 extends in the axial direction at the part of the end side joining part 72 in which the linear joining member 7 passes through the two through holes 63.

The joining state with the linear joining member 7 at the end side joining part 72 is not limited thereto. For example, as shown in FIG. 10, a linear joining member 7A passes through the two through holes 63, and the end part of the linear joining member 7A may be crossed and tied to form a figure 8, thereby joining the biodegradable fibers constituting the polygonal annular portions 6 adjacent to each other in the axial direction.

According to the stent 1A of the second embodiment, the same effects as those of the stent 1 of the first embodiment can be achieved. Specifically, according to the second embodiment, the biodegradable fibers constituting the polygonal annular portions 6 adjacent to each other in the axial direction J1 are joined to each other by the linear joining member 7 passing through the two through holes 63, whereby the radial bulkiness of the stent 1 can be reduced. Therefore, for example, when the stent 1 is placed in a gastrointestinal tract with a stent delivery system, it is possible to improve the storability in the stent delivery system.

In the present embodiment, one linear joining member 7 joins the biodegradable fibers constituting the polygonal annular portions 6 adjacent to each other in the axial direction of the stent 1 by passing through the two through holes 63 and crossing. The two through holes 63 are joined at each of a plurality of locations along the axial direction of the stent 1. Thus, since the linear joining member 7 does not need to be composed of a plurality of members, handling becomes easy, and the biodegradable fibers constituting the adjacent polygonal annular portions 6 can be easily joined.

In the first embodiment, the two through holes 23 are joined to each other with the joining member 3 having a short length and end parts processed by melting with heat. On the other hand, in the second embodiment, since one linear joining member 7 joins
the two through holes 63 at each of a plurality of locations along the axial direction of the stent 1, the tensile strength when the biodegradable fiber constituting the polygonal annular portion 6 is pulled can be improved.

### [Example]

Here, the present invention will be specifically described with reference to an example, but the present invention is not limited to the following example. Experiments were conducted in which the stents of the following example and comparative example were each stored in a stent delivery system. As shown in FIG. 1, the stents of both the example and the comparative example were each formed by connecting three polygonal annular portions 2 in an axial direction J. The polygonal annular portion 2 was formed of a PDO fiber (fiber diameter: 0.40 mm to 0.499 mm), and had a number of mountains of three. The stent diameter was 18 mm. As shown in FIG. 1, in a stent 1 of the example, adjacent polygonal annular portions 2 were connected to each other with a joining member 3. In the comparative example, bent parts of adjacent polygonal annular portions 2 facing each other were inserted into a tube and fixed with an adhesive to connect the adjacent polygonal annular portions 2.

Experiments were conducted using the stents of the example and comparative example to store them in a stent delivery system. Stents are generally stored in the following stent delivery system and placed inside the gastrointestinal tract. For example, the stent delivery system includes a cylindrical outer sheath member (outer cylinder) (not shown) inserted into the gastrointestinal tract, and a pusher member (not shown). A stent can be stored inside the outer sheath member. The stent is disposed inside the outer sheath member in a reduced diameter state (extended state in an axially direction). In this state, the stent is pushed out from the distal end side of the outer sheath member by the pusher member disposed on the proximal end side inside the outer sheath member. The stent is pushed out from the distal end side of the outer sheath member, expanded in diameter inside the gastrointestinal tract, and placed inside the gastrointestinal tract.

Using such a stent delivery system, when the inner diameter of the outer sheath member was 3.1 mm, experiments were conducted to store the stents of the example and comparative example inside the outer sheath member. When the stent of the example was used, the radial bulkiness was reduced, and the stent could be stored inside the outer sheath member disposed inside the gastrointestinal tract. When the stent of the comparative example was used, the stent could not be stored inside the outer sheath member disposed inside the gastrointestinal tract. From the above results, according to the present invention, it is possible to improve the storability in a stent delivery system.

### EXPLANATION OF REFERENCE NUMERALS

1 stent
2 polygonal annular portion
3 joining member
4 joining member
23 through hole
25 one end part
26 the other end part
27 through hole
J axial direction

## Claims

1. A stent (1A) formed of a linear member and in a cylindrical shape having a plurality of openings, the stent (1A) extending in an axial direction (J1) and being deformable from a reduced diameter state to an expanded diameter state,
the stent (1A) being formed by connecting a plurality of polygonal annular portions (6) each formed in a polygonal annular shape when viewed in an axial direction (J1), in a state of being bent or curved in the axial direction (J1), side by side in the axial direction (J1),
the linear members constituting the polygonal annular portions (6) adjacent to each other in the axial direction (J1) respectively having through holes (63) passing through in the axial direction (J1), the linear members being connected to each other with a joining member (7) passing through two of the through holes (63) of the linear members constituting the polygonal annular portions (6) adjacent to each other,
wherein the joining members (7) extend linearly and are respectively provided at a plurality of locations apart from each other in a circumferential direction of the stent (1A), and **characterized in that**
wherein one of the joining members (7) joins the linear members constituting the polygonal annular portions (6) adjacent to each other in the axial direction (J1) of the stent (1A) by passing through the two through holes (63) and crossing, and the two through holes (63) are joined to each other at each of a plurality of locations along the axial direction (J1) of the stent (1A).

2. The stent (1A) according to claim 1, wherein the joining member (7) passes through the two through holes (63) and extends in the axial direction (J1).

3. The stent (1A) according to claim 1 or 2, wherein at least one selected from the linear member constituting the stent (1A) and the joining member (7) is formed of a biodegradable fiber.

4. The stent (1A) according to any one of claims 1 to 3, wherein the through holes (63) are formed with a needle or a laser.

5. The stent (1A) according to any one of claims 1 to 4, wherein the joining member (7) is formed in a linear shape or a ring shape.

## Patentansprüche

1. Stent (1A), der aus einem linearen Element und in einer zylindrischen Form mit einer Vielzahl von Öffnungen gebildet ist, wobei sich der Stent (1A) in einer axialen Richtung (J1) erstreckt und von einem Zustand mit reduziertem Durchmesser zu einem Zustand mit erweiterten Durchmesser verformbar ist,
wobei der Stent (1A) gebildet wird, indem eine Vielzahl von polygonalen ringförmigen Abschnitten (6), die bei Betrachtung in der axialen Richtung (J1) jeweils in einer polygonalen ringförmigen Form ausgebildet sind, in einem in der axialen Richtung (J1) gebogenen oder gekrümmten Zustand nebeneinander in der axialen Richtung (J1) miteinander verbunden werden,
wobei die linearen Elemente, welche die in der axialen Richtung (J1) aneinander angrenzenden polygonalen ringförmigen Abschnitte (6) bilden, jeweils Durchgangslöcher (63) haben, die in der axialen Richtung (J1) verlaufen, wobei die linearen Elemente mit einem Verbindungselement (7) miteinander verbunden sind, das durch zwei der Durchgangslöcher (63) der die aneinander angrenzenden polygonalen ringförmigen Abschnitte (6) bildenden linearen Elemente verläuft,
wobei sich die Verbindungselemente (7) linear erstrecken und jeweils an einer Vielzahl von in einer Umfangsrichtung des Stents (1A) voneinander beabstandeten Stellen vorgesehen sind, und **dadurch gekennzeichnet, dass**
eines der Verbindungselemente (7) die linearen Elemente, welche die in der axialen Richtung (J1) des Stents (1A) aneinander angrenzenden polygonalen ringförmigen Abschnitte (6) bilden, verbindet, indem es durch die zwei Durchgangslöcher (63) verläuft und sie durchquert, und dass die zwei Durchgangslöcher (63) an jeder aus einer Vielzahl von Stellen entlang der axialen Richtung (J1) des Stents (1A) miteinander verbunden sind.

2. Stent (1A) nach Anspruch 1, wobei das Verbindungselement (7) durch die zwei Durchgangslöcher (63) verläuft und sich in der axialen Richtung (J1) erstreckt.

3. Stent (1A) nach Anspruch 1 oder 2, wobei das den Stent (1A) bildende lineare Element und/oder das Verbindungselement (7) aus einer biologisch abbaubaren Faser gebildet sind.

4. Stent (1A) nach einem der Ansprüche 1 bis 3, wobei die Durchgangslöcher (63) mit einer Nadel oder einem Laser gebildet sind.

5. Stent (1A) nach einem der Ansprüche 1 bis 4, wobei das Verbindungselement (7) in einer linearen Form oder einer Ringform ausgebildet ist.

## Revendications

1. Stent (1A) formé d'un élément linéaire avec une forme cylindrique et comprenant une pluralité d'ouvertures, le stent (1A) s'étendant dans une direction axiale (J1) et pouvant être déformé d'un état de diamètre réduit à un état de diamètre élargi,
le stent (1A) étant formé en connectant une pluralité de sections annulaires polygonales (6), formées dans une forme annulaire polygonale lorsqu'elles sont vues dans la direction axiale (J1), côte à côte dans la direction axiale (J1) dans un état courbé ou incurvé dans la direction axiale (J1),
les éléments linéaires, formant les sections annulaires polygonales (6) adjacentes dans la direction axiale (J1), comportant chacun des trous traversants (63) qui s'étendent dans la direction axiale (J1), les éléments linéaires étant reliés entre eux par un élément de liaison (7) qui traverse deux des trous traversants (63) des éléments linéaires formant les sections annulaires polygonales (6) adjacentes,
les éléments de liaison (7) s'étendant linéairement et étant prévus chacun en une pluralité d'emplacements espacés les uns des autres dans une direction circonférentielle du stent (1A), et **caractérisé en ce que**
l'un des éléments de liaison (7) relie les éléments linéaires qui forment les sections annulaires polygonales (6) adjacentes dans la direction axiale (J1) du stent (1A), en passant à travers les deux trous traversants (63) et en les traversant, et **en ce que** les deux trous traversants (63) sont reliés l'un à l'autre en plusieurs emplacements le long de la direction axiale (J1) du stent (1A).

2. Le stent (1A) selon la revendication 1, sachant que l'élément de liaison (7) traverse les deux trous traversants (63) et s'étend dans la direction axiale (J1).

3. Le stent (1A) selon la revendication 1 ou 2, sachant que l'élément linéaire formant le stent (1A) et/ou l'élément de liaison (7) sont constitués d'une fibre biodégradable.

4. Le stent (1A) selon l'une des revendications 1 à 3, sachant que les trous traversants (63) sont réalisés au moyen d'une aiguille ou d'un laser.

5. Le stent (1A) selon l'une des revendications 1 à 4, sachant que l'élément de liaison (7) est réalisé sous une forme linéaire ou une forme annulaire.
